(19) Europäisches Patentamt
European Patent Office
Office européen des brevets

(11) Veröffentlichungsnummer: **0 041 177**
**B1**

(12) # EUROPÄISCHE PATENTSCHRIFT

(45) Veröffentlichungstag der Patentschrift: **10.10.84**

(51) Int. Cl.³: **C 07 D 253/06** // A01N43/64

(21) Anmeldenummer: **81103822.3**

(22) Anmeldetag: **19.05.81**

(54) **Verfahren zur Herstellung von 4-Amino-6-tert.butyl-3-mercapto-1,2,4-triazin-5-on.**

(30) Priorität: **29.05.80 DE 3020370**

(43) Veröffentlichungstag der Anmeldung: **09.12.81 Patentblatt 81/49**

(45) Bekanntmachung des Hinweises auf die Patenterteilung: **10.10.84 Patentblatt 84/41**

(84) Benannte Vertragsstaaten: **AT BE CH FR GB IT LI NL**

(56) Entgegenhaltungen:
**DE - A - 2 165 554**
**DE - A - 2 726 016**
**DE - A - 2 732 797**
**DE - A - 2 733 180**
**DE - A - 2 856 750**
**FR - A - 1 547 854**

**CHEMISCHE BERICHTE; Band 97, veröffentlicht in 1964, Seiten 2173-2179 Weinheim, DE. A. DORNOW et al.: "Über 1.2.4-Triazine, I. Darstellung einiger neuer s-Traizolo (3.2-c)-as-triazine"**
**JOURNAL OF THE AMERICAN CHEMICAL SOCIETY, Band 70, veröffentlicht in 1948, Seiten 40454050 Columbus, Ohio, U.S.A. J.J. RITTER et al.: "A new reaction of nitriles. I. Amides from alkenes and mononitriles"**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Greifenhagenstrasse 9, D-6450 Hanau 9 (DE)**
Erfinder: **Lehmann, Bernd, Dr., Flurstrasse 5, D-6463 Freigericht (DE)**
Erfinder: **Klenk, Herbert, Dr., Grünaustrasse 19, D-6450 Hanau 9 (DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Gegenstand der Erfindung ist ein Verfahren zur Herstellung von 4-Amino-6-tert.butyl-3-mercapto-1,2,4-triazin-5-on (I),

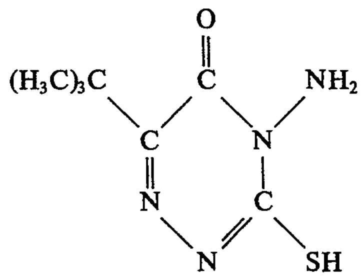

(I)

bei dem in einer ersten Reaktionsstufe Pivaloylcyanid (II)

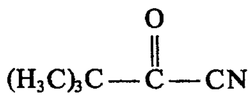

(II)

in Gegenwart von Essigsäure und Schwefelsäure mit Isobuten umgesetzt und in einer letzten Reaktionsstufe mittels Thiocarbohydrazid (III)

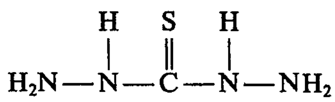

(III)

das 4-Amino-6-tert.butyl-3-mercapto-1,2,4-triazin-5-on (I) gebildet wird.

4-Amino-6-tert.butyl-3-mercapto-1,2,4-triazin-5-on ist ein wichtiges Zwischenprodukt bei der Synthese des bekannten Herbicids 4-Amino-6-tert.butyl-3-methylmercapto-1,2,4-triazin-5-on.

Aus der DE-AS 2 733 180 ist bereits ein Verfahren zur Herstellung von 4-Amino-6-tert.butyl-3-mercapto-1,2,4-triazin-5-on bekannt. In einer ersten Reaktionsstufe wird Pivaloylcyanid in Gegenwart von Essigsäure und 100%iger Schwefelsäure mit Isobuten umgesetzt und das bei dieser Umsetzung gebildete Trimethylbrenztraubensäure-tert.butylamid isoliert. Anschließend wird das Trimethylbrenztraubensäure-tert.butylamid durch Erhitzen mit wäßriger Salzsäure verseift und die gebildete Trimethylbrenztraubensäure isoliert. In einer letzten Reaktionsstufe wird dann schließlich in wäßrig-alkoholischer Lösung die Trimethylbrenztraubensäure mit Thiocarbohydrazid zu dem 4-Amino-6-tert.butyl-3-mercapto-1,2,4-triazin-5-on kondensiert. Bei der technischen Durchführung des bekannten Verfahrens ist jedoch der hohe Anfall an mit anorganischen und organischen Verbindungen verschiedener Art belasteten Abwässern sehr störend. Denn eine wirtschaftliche Aufarbeitung dieser Abwässer ist aufgrund ihrer komplexen Zusammensetzung kaum möglich. Außerdem wirft die Verwendung von Salzsäure Korrosionsprobleme auf. Schließlich ist es auch nicht möglich, das eingesetzte Isobuten wenigstens teilweise zurückzugewinnen.

Das erfindungsgemäße Verfahren ist nun dadurch gekennzeichnet, daß man die Umsetzung des Pivaloylcyanids (II) mit Isobuten in Gegenwart von 1,4 bis 1,7 Mol pro Mol eingesetzten Pivaloylcyanids Schwefelsäure einer Konzentration zwischen 93 und 100 Gewichtsprozent vornimmt, das bei dieser Umsetzung erhaltene Reaktionsgemisch mit Wasser und gegebenenfalls weiterer Essigsäure in solcher Menge versetzt, daß es, jeweils pro Mol eingesetzten Pivaloylcyanids, 50 bis 220 g Wasser und insgesamt 150 bis 850 g Essigsäure enthält mit der Maßgabe, daß das Gewichtsverhältnis von Wasser zu Essigsäure zwischen 0,1 und 1,0 liegt, anschließend so lange zum Sieden erhitzt, bis dünnschichtchromatographisch kein Trimethylbrenztraubensäure-tert.butylamid

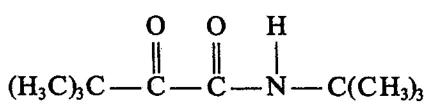

(IV)

mehr nachweisbar ist, das Reaktionsgemisch gegebenenfalls mit so viel weiterem Wasser verdünnt, daß das Gewichtsverhältnis von Wasser zu Essigsäure zwischen 0,4 und 2,0 liegt, und mit mindestens der dem durch cerimetrische Titration bestimmten Gehalt an Trimethylbrenztraubensäure

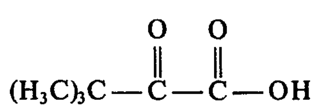

(V)

äquivalenten Menge Thiocarbohydrazid (III) das 4-Amino-6-tert.butyl-3-mercapto-1,2,4-triazin-5-on (I) ausfällt.

Analog gebaute Verbindungen, bei denen an Stelle der 6-tert.Butylgruppe eine 1-Methyl-cyclopropylgruppe- oder eine 2,2-Dichlor-1-methyl-cyclopropylgruppe oder aber andere Alkyl-, Aralkyl- oder Arylreste stehen, sind bereits nach der von A. Dornow beschriebenen Methode (Chemische Berichte Bd. 97, 1964 Seiten 2173–2179) bekannt geworden (DE-OS 2 732 797, DE-OS 2 856 750, DE-OS 2 726 016, FP-PS 1 547 854).

Nach diesen Verfahren können jedoch die weiter unten dargelegten Vorteile nicht erreicht werden.

Es ist auch bekannt, das 4-Amino-6-tert.butyl-3-methylmercapto-1,2,4-triazin-5-on durch Umsetzung von Pivaloylchlorid mit einem Isonitril, Hydrolysieren des gebildeten Imidchlorids zum $\alpha$-Ketocarbonsäureamid und Weiterumsetzung des Amids mit Thiocarbohydrazid in einem polaren Lösungsmittel herzustellen. Bei diesem Verfahren fällt nachteilig ins Gewicht, daß der Umgang mit den sehr übelriechenden und zudem sehr kostspieligen Isonitrilen erforderlich ist. Außerdem kann auch diesem Verfahren die 3-Mercapto-Verbindung nur in Ausbeuten von etwa 60 bzw. 79% erhalten werden.

Das erfindungsgemäße Verfahren wird ohne Isolierung von Zwischenprodukten als »Eintopf-Verfahren« durchgeführt und ist infolgedessen technisch sehr einfach. Der Anfall an Abwässern ist erheblich geringer als bei dem bekannten Verfahren. Da ferner als Lösungsmittel nur Wasser und Essigsäure verwendet werden, ist die Aufarbeitung der Abwässer sehr viel leichter zu bewerkstelligen. Außerdem kann zumindest ein Teil des eingesetzten Isobutens auf einfache Weise in wieder einsetzbarer Form zurückgewonnen werden. Bei geeigneter Wahl der Reaktionsbedingungen können ferner erheblich höhere Ausbeuten, bezogen auf das eingesetzte Pivaloylcyanid, erzielt werden.

Besonders vorteilhaft im Hinblick auf die Möglichkeit zur Rückgewinnung des eingesetzten Isobutens und eine hohe Ausbeute ist es, wenn man das bei der Umsetzung des Pivaloylcyanids mit Isobuten erhaltene Reaktionsgemisch mit Wasser gegebenenfalls weiterer Essigsäure in solcher Menge versetzt, daß es, jeweils pro Mol eingesetzten Pivaloylcyanids, 100 bis 200 g Wasser und insgesamt 250 bis 450 g Essigsäure enthält mit der Maßgabe, daß das Gewichtsverhältnis von Wasser zu Essigsäure zwischen 0,25 und 0,55 liegt.

Die bei der Umsetzung des Pivaloylcyanids mit Isobuten eingesetzte Menge an Essigsäure ist für den Ablauf der Reaktion nicht entscheidend, jedoch verwendet man zweckmäßigerweise pro Mol eingesetzten Pivaloylcyanids 100 bis 600 g, vorzugsweise 150 bis 250 g, Essigsäure. Die Schwefelsäure wird pro Mol eingesetzten Pivaloylcyanids in einer Menge von 1,4 bis 1,7 Mol eingesetzt. Auch das Isobuten wird zweckmäßigerweise im Überschuß angewandt, beispielsweise in einer Menge von 1,7 bis 2 Mol pro Mol eingesetzten Pivaloylcyanids. Die Temperatur während der Umsetzung kann innerhalb weiter Grenzen variiert werden. Bevorzugt werden Temperaturen zwischen −20 und +50°C, insbesondere zwischen 0 und +20°C.

Das nach beendeter Umsetzung mit Wasser und gegebenenfalls weiterer Essigsäure versetzte Reaktionsgemisch wird anschließend so lange auf Rückflußtemperatur erhitzt, bis darin dünnschichtchromatographisch kein Trimethylbrenztraubensäure-tert.butylamid mehr nachweisbar ist. Die dünnschichtchromatographische Prüfung erfolgt in der Weise, daß eine Probe des Reaktionsgemisches auf eine DC-Fertigplatte (Kieselgel 60 $F_{254}$, Schichtdicke 0,25 mm; Hersteller: E. Merck, Art. Nr. 5714) aufgetragen und kurz getrocknet wird. Dann wird mit Ether als Laufmittel entwickelt. Nach dem Verdunsten des Lösungsmittels erkennt man etwa noch vorhandenes Trimethylbrenztraubensäure-tert.butylamid im UV-Licht der Wellenlänge 254 nm als einen Fleck mit dem $R_f$-Wert 0,67.

Während des Erhitzens entweicht aus dem Reaktionsgemisch abgespaltenes Isobuten, das leicht in einer Kühlfalle aufgefangen werden kann. Es kann durch Durchleiten eines schwachen Stickstoffstromes durch das Reaktionsgemisch besonders rasch und gründlich aus dem Reaktionsgemisch entfernt werden.

Anschließend wird das Reaktionsgemisch gegebenenfalls mit so viel weiterem Wasser verdünnt, daß das Gewichtsverhältnis von Wasser zu Essigsäure zwischen 0,4 und 2,0, vorzugsweise zwischen 0,65 und 1,45, liegt, mit mindestens der dem durch cerimetrische Titration bestimmten Gehalt an Trimethylbrenztraubensäure äquivalenten Menge Thiocarbohydrazid versetzt und auf Raumtemperatur abgekühlt. Dabei fällt das gewünschte 4-Amino-6-tert.butyl-3-mercapto-1,2,4-triazin-5-on in dünnschichtchromatographisch reiner Form aus.

Um in dieser Reaktionsstufe die Bildung von unerwünschten und die Gesamtausbeute vermindernden Nebenprodukten zu unterdrücken, hat es sich als vorteilhaft erwiesen, das vor der Zugabe des Thiocarbohydrazids zuzusetzende Wasser in Form einer wäßrigen Base, z. B. als wäßrigen Ammoniak oder als Natronlauge, einzubringen, wobei zweckmäßigerweise der Gehalt an Base der noch im Reaktionsgemisch vorhandenen Schwefelsäuremenge äquivalent sein sollte. Es kann aber natürlich auch ein geringer Überschuß an Base angewandt werden.

Diese Maßnahme begünstigt gleichzeitig die extraktive Rückgewinnung der Essigsäure aus dem nach der Abtrennung des 4-Amino-6-tert.butyl-3-mercapto-1,2,4-triazin-5-ons verbleibenden Abwasser, da in Abwesenheit von freier Schwefelsäure auch hydrolyseempfindliche Extraktionsmittel, wie Essigester, erfolgreich und ohne nennenswerte Extraktionsmittelverluste eingesetzt werden können. Alternativ kann die Essigsäure auch destillativ aus dem Abwasser zurückgewonnen werden. Wurde Ammoniak zur Neutralisation der Schwefelsäure verwendet, so kann der nun verbleibende Rückstand

auch noch auf Ammoniumsulfat, z. B. für Düngemittelzwecke, aufgearbeitet werden.

Die Erfindung soll durch die nachfolgenden Beispiele näher erläutert werden. Sofern nicht anders angegeben, bedeuten die Prozentangaben Gewichtsprozente.

## Beispiel 1

111 g (1 Mol) Pivaloylcyanid werden in 200 ml Eisessig gelöst und bei 0 bis +5° C mit 152 g (1,5 Mol) 97%iger Schwefelsäure versetzt. Innerhalb von 2 Stunden leitet man 112,2 g (2 Mol) Isobuten bei 0 bis +5° C ein. Im Anschluß an die einstündige Nachreaktion bei 20° C werden bei der gleichen Temperatur 200 ml Eisessig und 108 g Wasser zugefügt. Das Gemisch wird in einem sehr langsamen Stickstoffstrom für 4 Stunden zum Sieden erhitzt, wobei 92 g (82% des Einsatzes) Isobuten entweichen, die in einer Kühlfalle aufgefangen werden. Man versetzt bei 80° C mit 100 g Wasser und anschließend mit 92,4 g (0,87 Mol) Thiocarbohydrazid. Es wird noch 10 Minuten bei 95 bis 100° C gerührt und dann abgekühlt. Durch Filtration isoliert man 162 g des Triazinons I vom Schmelzpunkt 208 – 210° C (81% der Theorie).

## Beispiel 2

Man verfährt wie im Beispiel 1, versetzt jedoch vor Zugabe des Thiocarbohydrazids statt mit 100 g Wasser mit 340 g (2 Mol) 10%iger wäßriger Ammoniaklösung und anschließend mit 93,4 g (0,88 Mol) Thiocarbohydrazid. Man isoliert 174 g (87% der Theorie) des Triazinons I vom Schmelzpunkt 209 – 212° C.

Aus dem Filtrat wird mit Essigsäureethylester die Essigsäure extrahiert und destillativ zurückgewonnen. Aus dem Rückstand der Extraktion lassen sich durch Einengen und Kristallisieren 194 g (98% der Theorie) Ammoniumsulfat gewinnen.

## Beispiel 3

111 g (1 Mol) Pivaloylcyanid werden in 200 ml Eisessig gelöst und bei 0 bis +5° C mit 152 g (1,5 Mol) 97%iger Schwefelsäure versetzt. Innerhalb von 2 Stunden leitet man 112,2 g (2 Mol) Isobuten bei 0 bis +5° C ein. Im Anschluß an die einstündige Nachreaktion bei 20° C werden bei gleicher Temperatur 200 ml Eisessig und 198 g Wasser zugefügt. Das Gemisch wird in einem sehr langsamen Stickstoffstrom für 12 Stunden zum Sieden erhitzt, wobei 97 g (86% des Einsatzes) Isobuten entweichen, die in einer Kühlfalle aufgefangen werden.

Man versetzt mit 236 g (2 Mol) einer 14,4%igen wäßrigen Ammoniaklösung und anschließend mit 98 g (0,92 Mol) Thiocarbohydrazid. Es wird noch 5 Minuten bei 100° C gerührt und dann abgekühlt. Durch Filtration isoliert man 182 g (91% der Theorie) Triazinon I vom Schmelzpunkt 209 – 212° C.

## Beispiele 4 bis 10

Es werden jeweils 111 g (1 Mol) Pivaloylcyanid in 210 g Eisessig gelöst und bei 0 bis +5° C mit 152 g (1,5 Mol) 97%iger Schwefelsäure versetzt. Innerhalb von 2 Stunden leitet man 112,2 g (2 Mol) Isobuten bei 0 bis +5° C ein. Im Anschluß an die einstündige Nachreaktion bei +20° C werden bei der gleichen Temperatur die in Tabelle 1 angegebenen Mengen an Essigsäure und Wasser zugefügt. Das Gemisch wird in einem sehr langsamen Stickstoffstrom so lange zum Sieden erhitzt, bis dünnschichtchromatographisch kein Trimethylbrenztraubensäure-tert.butylamid mehr nachweisbar ist. Das entweichende Isobuten wird in einer Kühlfalle aufgefangen und ausgewogen. Durch cerimetrische Titration wird der Gehalt des Reaktionsgemisches an Trimethylbrenztraubensäure bestimmt.

Man versetzt mit 236 g (2 Mol) einer 14,4%igen wäßrigen Ammoniaklösung und anschließend mit 102% der der Trimethylbrenztraubensäure äquivalenten Thiocarbohydrazid-Menge.

Man erhitzt auf 100° C, rührt noch ca. 5 bis 10 Minuten bei dieser Temperatur und läßt dann abkühlen. Das Triazinon I wird in dünnschichtchromatographisch reiner Form durch Filtration isoliert.

Tabelle 1

| Beispiel | Essigsäurezusatz vor Verseifung [g] | Gesamt Essig säure [g] | Wasserzusatz [g] | Gewichtsverhältnis Wasser/Gesamt Essigsäure | Zurückgewonnenes Isobuten [% d. Einsatzes] | Ausbeute an Triazinon I [% der Theorie] |
|---|---|---|---|---|---|---|
| 4 | 0 | 210 | 63 | 0,30 | 30 | 62 |
| 5 | 0 | 210 | 108 | 0,51 | 69 | 83 |
| 6 | 0 | 210 | 198 | 0,94 | 48 | 67 |
| 7 | 210 | 420 | 63 | 0,15 | 16 | 72 |
| 8 | 210 | 420 | 108 | 0,26 | 82 | 87 |
| 9 | 210 | 420 | 198 | 0,47 | 86 | 91 |
| 10 | 630 | 840 | 108 | 0,13 | 17 | 86 |

Vergleichsversuch 1

Es wurde verfahren wie bei den Beispielen 4 bis 6, jedoch wurden nach der Umsetzung des Pivaloylcyanids mit dem Isobuten 378 g Wasser zugesetzt. Das Gewichtsverhältnis Wasser/Gesamt-Essigsäure betrug demnach 1,80. Während 12stündigen Erhitzens am Rückfluß entwich kein Isobuten aus dem Reaktionsgemisch. Nach dieser Zeit ergab die cerimetrische Titration einen Gehalt an Trimethylbrenztraubensäure von nur 5,85 g (4,5% der Theorie).

Vergleichsversuch 2

Es wurde verfahren wie bei den Beispielen 7 bis 9, jedoch wurden nach der Umsetzung des Pivaloylcyanids mit dem Isobuten 378 g Wasser zugesetzt. Das Gewichtsverhältnis Wasser/Gesamt-Essigsäure betrug demnach 0,90. Während 12stündigen Erhitzens am Rückfluß entwichen nur etwa 5,6 g (5% des Einsatzes) Isobuten aus dem Reaktionsgemisch. Nach dieser Zeit ergab die cerimetrische Titration einen Gehalt an Trimethylbrenztraubensäure von nur 26 g (20% der Theorie).

Beispiele 11 bis 14

Es werden jeweils 111 g (1 Mol) Pivaloylcyanid in 210 g Eisessig gelöst und bei 0 bis +5°C mit 1,5 Mol Schwefelsäure versetzt, die den in Tabelle 2 angegebenen Wassergehalt hat. Innerhalb von 2 Stunden leitet man 112,2 g (2 Mol) Isobuten bei 0 bis +5°C ein. Im Anschluß an die einstündige Nachreaktion bei +20°C werden bei der gleichen Temperatur 210 g Eisessig und die in Tabelle 2 angegebene Wassermenge zugefügt. Die Wassermengen werden so gewählt, daß die Summe der durch die Schwefelsäure eingebrachten und der zugesetzten Wassermenge gleich ist.

Das Gemisch wird in einem langsamen Stickstoffstrom für $4^1/_2$ Stunden zum Sieden erhitzt.

Durch cerimetrische Titration wird der Gehalt des Reaktionsgemisches an Trimethylbrenztraubensäure bestimmt.

Man versetzt mit 340 g 10%iger wäßriger Ammoniak-Lösung und anschließend mit 102% der der Trimethylbrenztraubensäure äquivalenten Thiocarbohydrazid-Menge.

Man erhitzt auf 95°C, rührt noch 10 Minuten bei dieser Temperatur und läßt dann abkühlen.

Das Triazinon I wird in dünnschichtchromatographsich reiner Form durch Filtration isoliert.

Tabelle 2

| Beispiel | Wassergehalt der Schwefelsäure in % | in g | Wasserzusatz in g | Wasser insgesamt in g | Ausbeute an Triazinon I % der Theorie |
|---|---|---|---|---|---|
| 11 | 7 | 11,1 | 101,5 | 112,6 | 82,5 |
| 12 | 5 | 7,7 | 104,9 | 112,6 | 84,5 |
| 13 | 3 | 4,6 | 108 | 112,6 | 87 |
| 14 | 0 | 0 | 112,6 | 112,6 | 84 |

**Patentansprüche**

1. Verfahren zur Herstellung von 4-Amino-6-tert.butyl-3-mercapto-1,2,4-triazin-5-on,

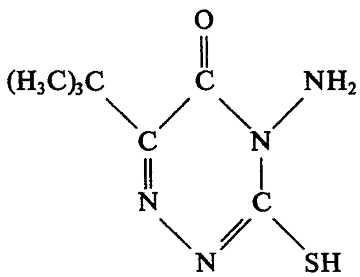

(I)

bei dem in einer ersten Reaktionsstufe Pivaloylcyanid

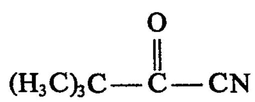

(II)

in Gegenwart von Essigsäure und Schwefelsäure mit Isobuten umgesetzt und in einer letzten Reaktionsstufe mittels Thiocarbohydrazid

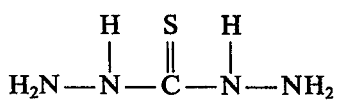

(III)

das 4-Amino-6-tert.butyl-3-mercapto-1,2,4-triazin-5-on (I) gebildet wird, dadurch gekennzeichnet, daß man die Umsetzung des Pivaloylcyanids (II) mit Isobuten in Gegenwart von 1,4 bis 1,7 Mol pro Mol eingesetzten Pivaloylcyanids Schwefelsäure einer Konzentration zwischen 93 und 100 Gewichtsprozent vornimmt, das bei dieser Umsetzung erhaltene Reaktionsgemisch mit Wasser und gegebenenfalls weiterer Essigsäure in solcher Menge versetzt, daß es, jeweils pro Mol eingesetzten Pivaloylcyanids, 50 bis 220 g Wasser und insgesamt 150 bis 850 g Essigsäure enthält, mit der Maßgabe, daß das Gewichtsverhältnis von Wasser zu Essigsäure zwischen 0,1 und 1,0 liegt, anschließend so lange zum Sieden erhitzt, bis dünnschichtchromatographisch kein Trimethylbrenztraubensäure-tert.butylamid

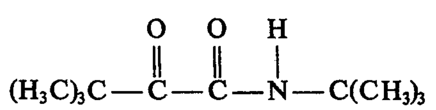

(IV)

mehr nachweisbar ist, das Reaktionsgemisch gegebenenfalls mit so viel weiterem Wasser verdünnt, daß das Gewichtsverhältnis von Wasser zu Essigsäure zwischen 0,4 und 2,0 liegt, und mit mindestens der dem durch cerimetrische Titration bestimmten Gehalt an Trimethylbrenztraubensäure

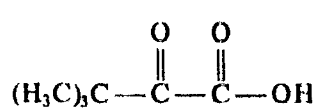

(V)

äquivalenten Menge Thiocarbohydrazid (III) das 4-Amino-6-tert.butyl-3-mercapto-1,2,4-triazin-5-on (I) ausfällt.

2. Verfahren nach Anspruch 1, dadurch gekennzeichnet, daß man das bei der Umsetzung des Pilvaloylcyanids mit Isobuten erhaltene Reaktionsgemisch mit Wasser und gegebenenfalls weiterer Essigsäure in solcher Menge versetzt, daß es, jeweils pro Mol eingesetzten Pivaloylcyanids, 100 bis 200 g Wasser und insgesamt 250 bis 450 g Essigsäure enthält, mit der Maßgabe, daß das Gewichtsverhältnis von Wasser zu Essigsäure zwischen 0,25 und 0,55 liegt.

3. Verfahren nach Anspruch 1 oder 2, dadurch gekennzeichnet, daß man das vor der Zugabe des Thiocarbohydrazids zuzusetzende Wasser in Form einer wäßrigen Base einbringt, deren Gehalt an Base der noch im Reaktionsgemisch vorhandenen Schwefelsäuremenge mindestens äquivalent ist.

4. Verfahren nach Anspruch 3, dadurch gekennzeichnet, daß man als wäßrige Base eine wäßrige Ammoniaklösung einsetzt.

## Claims

1. A process for the production of 4-amino-6-tert.butyl-3-mercapto-1,2,4-triazin-5-one,

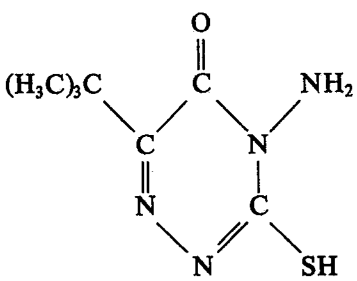

(I)

in which, in a first reaction stage, pivaloylcyanide

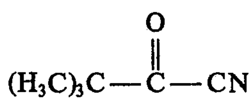

(II)

is reacted with isobutene in the presence of acetic acid and sulphuric acid, and in a final reaction stage 4-amino-6-tert.butyl-3-mercapto-1,2,4-triazin-5-one (I) is formed by means of thiocarbohydrazide

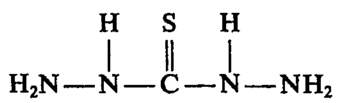

(III)

characterised in that the reaction of pivaloylcyanide (II) with isobutene is carried out in the presence of from 1.4 to 1.7 mols of sulphuric acid having a concentration of from 93 to 100%, by weight, per mol of pivaloylcyanide which is used, the mixture which is obtained during this reaction is mixed with water and optionally more acetic acid in a quantity, such that the reaction mixture contains from 50 to 220 g of water and in total from 150 to 850 g of acetic acid, in each case per mol of pivaloylcyanide which is used, with the condition that the weight ratio of water to acetic acid is from 0.1 to 1.0, and the reaction mixture is subsequently heated to boiling point, until, no more trimethyl-pyroracemic acid-tert.butyl amide

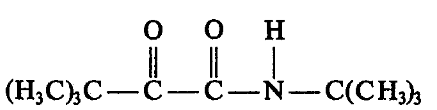

(IV)

may be detected, by means of thin-layer chromatography, the reaction mixture is optionally diluted with more water until the weight ratio of water to acetic acid is from 0.4 to 2.0, and the 4-amino-6-tert.butyl-3-mercapto-1,2,4-triazin-5-one (I) precipitates with at least the quantity of thiocarbohydrazide (III) which is equivalent to the content of trimethyl pyroracemic acid

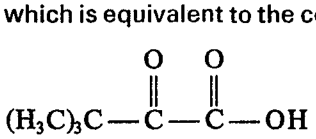

(V)

which is determined by means of cerimetric titration.

2. A process according to claim 1, characterised in that the reaction mixture which is obtained by reacting pivaloylcyanide with isobutene is mixed with water and optionally further acetic acid in a quantity such that the reaction mixture contains from 100 to 200 g of water and in total from 250 to 450 g of acetic acid, in each case per mol of pivaloylcyanide which is used, on condition that the weight ratio of water to acetic acid is from 0.25 to 0.55.

3. A process according to claim 1 or 2, characterised in that the water which is to be added before the addition of thiocarbohydrazide is introduced in the form of an aqueous base, the base content of which is at least equivalent to the quantity of sulphuric acid which is still present in the reaction mixture.

4. A process according to claim 3, characterised in that an aqueous ammonia solution is used as the aqueous base.

**Revendications**

1. Procédé de préparation du 4-amino-6-tert.butyl-3-mercapto-1,2,4-triazin-5-one,

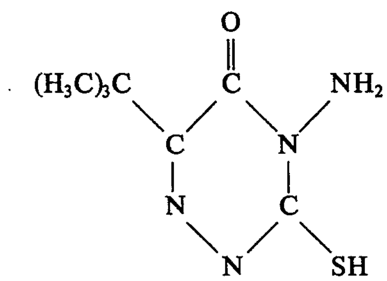

(I)

selon lequel, dans une première étape réactionnelle, on fait réagir le cyanure de l'acide pivalique,

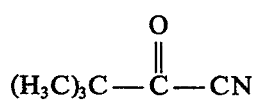

(II)

avec de l'isobutène, en présence d'acide acétique et d'acide sulfurique, et dans une dernière étape réactionnelle, le 4-amino-6-tert.butyl-3-mercapto-1,2,4-triazine-5-one (I) est formé à l'aide du thiocarbohydrazide:

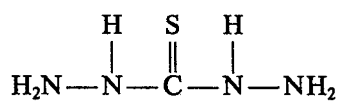

(III)

procédé caractérisé en ce que, l'on effectue la réaction du cyanure de l'acide pivalique (II) avec l'isobutène en présence de 1,4 à 1,7 mole, par mole de cyanure de l'acide pivalique mis en oeuvre, d'acide sulfurique à une concentration comprise entre 93 et 100% en poids, on ajoute au mélange réactionnel obtenu par cette réaction, de l'eau et éventuellement encore de l'acide acétique, en une quantité telle que le mélange réactionnel contienne chaque fois, par mole de cyanure de l'acide pivalique mis en oeuvre, 50 à 220 g d'eau et en tout 150 à 850 g d'acide acétique, avec cette condition que le rapport en poids de l'eau à l'acide acétique soit compris entre 0,1 et 1, ensuite on chauffe à l'ébullition jusqu'au moment où la chromatographie en couche mince n'indique plus la présence de tert.butylamide triméthyl-pyruvique,

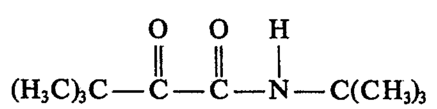

(IV)

on dilue éventuellement le mélange réactionnel avec une nouvelle quantité d'eau telle que le rapport en poids de l'eau à l'aide acétique soit compris entre 0,4 et 2, et l'on précipite le 4-amino-6-tert.butyl-3-mercapto-1,2,4-triazin-5-one (I) avec une quantité de thiocarbohydrazide (III) au moins équivalente à la teneur en acide triméthylpyruvique

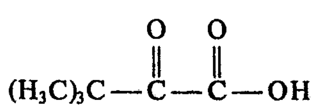

(V)

déterminée par dosage cérimétrique.

2. Procédé selon la revendication 1, caractérisé en ce que l'on ajoute au mélange réactionnel obtenu en faisant réagir le cyanure de l'acide pivalique avec l'isobutène, de l'eau et éventuellement encore de l'acide acétique, dans une proportion telle que pour 1 mole de cyanure de l'acide pivalique mis en oeuvre, le mélange contienne toujours de 100 à 200 g d'eau et au total de 250 à 450 g d'acide acétique, avec cette condition que le rapport en poids de l'eau à l'acide acétique soit compris entre 0,25 et 0,55.

3.Procédé selon l'une des revendications 1 ou 2, caractérisé en ce que l'on introduit l'eau devant être ajoutée avant l'addition de thiocarbohydrazide, sous la forme d'une base aqueuse, dont la teneur en base est au moins équivalente à la quantité d'acide sulfurique encore présente dans le mélange réactionnel.

4. Procédé selon la revendication 3, caractérisé en ce que comme base aqueuse, on utilise une solution hydroammoniacale.